Europäisches Patentamt

European Patent Office

Office européen des brevets

(11) Numéro de publication: **0 312 475**
**A1**

# DEMANDE DE BREVET EUROPEEN

(21) Numéro de dépôt: **88420348.0**

(22) Date de dépôt: **14.10.88**

(51) Int. Cl.⁴: **B 41 F 5/20**
**G 01 N 33/34**

(30) Priorité: **16.10.87 FR 8714565**

(43) Date de publication de la demande:
**19.04.89 Bulletin 89/16**

(84) Etats contractants désignés:
**AT BE CH DE ES FR GB GR IT LI LU NL SE**

(71) Demandeur: **CENTRE TECHNIQUE DE L'INDUSTRIE DES PAPIERS, CARTONS ET CELLULOSES.**
**Domaine Universitaire**
**F-38000 Grenoble (FR)**

(72) Inventeur: **Chiodi, René**
**Chemin de Pressembois Venon**
**F-38610 Gières (FR)**

(74) Mandataire: **Laurent, Michel et al**
**Cabinet LAURENT et GUERRE 20, rue Louis Chirpaz B.P. 32**
**F-69131 Ecully Cédex (FR)**

(54) Cliché d'impression pour tester l'aptitude à l'impression flexographique d'un papier, d'un carton ou d'un matériau analogue en feuille.

(57) Cliché souple (2) d'impression, pour tester l'aptitude d'un papier, d'un carton ou d'un matériau analogue en feuille à l'impression flexographique, destiné à être monté sur un dispositif d'impression du type comprenant :
. un rouleau doseur (6) tramé, interchangeable,
. un moyen (8) pour déposer de l'encre liquide (7) sur ledit rouleau (6) et retirer l'excès d'encre (7) déposé ;
. un rouleau porte-cliché (1) moteur, destiné à recevoir ledit cliché (2) et à venir au contact tangentiel avec le dit rouleau doseur (6), et à tourner autour de son axe pour définir un sens de défilement F de l'impression ;
. une molette cylindrique (9), destinée à recevoir un échantillon à tester, et à venir au contact tangentiel avec le cliché (2) encré ;
**caractérisé** en ce que le dit cliché (2) comprend au moins un aplat, et au moins une surface tramée dont la linéature varie de façon continue dans le sens de défilement FD de l'impression.

FIG.1

## Description

### CLICHE D'IMPRESSION POUR TESTER L'APTITUDE A L'IMPRESSION FLEXOGRAPHIQUE D'UN PAPIER, D'UN CARTON OU D'UN MATERIAU ANALOGUE EN FEUILLE

L'invention concerne un cliché d'impression pour tester l'aptitude à l'impression flexographique d'un papier, d'un carton ou d'un matériau analogue en feuille et accessoirement déterminer les conditions optimales d'impression.

Si dans la suite de la description et des revendications, on se réfère le plus généralement à l'impression d'une feuille de papier, il va de soi que l'invention n'est nullement limitée à ce mode d'exécution préféré, puisque comme déjà dit on peut faire appel à d'autres matériaux analogues en feuille, tel que le carton.

Comme on le sait, la "flexographie" est une technique d'impression qui connait actuellement un fort développement, qui consiste à utiliser un cliché transfert dont les caractères en relief sont en un matériau souple, et à encrer ce cliché par report d'un film d'encre liquide déposé au moyen d'un rouleau doseur en contact avec le cliché.

A l'heure actuelle, il n'existe pas de test simple, rapide et significatif qui permette de reproduire en laboratoire les conditions industrielles exactes de l'impression flexographique et de prévoir le comportement des papiers lor de leur impression sur de telles rotatives. L'imprimeur est donc obligé de faire tous ses réglages, et en particulier de choisir les trames du rouleau doseur d'encre et du cliché flexographique, directement sur la rotative. Cela implique soit des résultats d'impression pas toujours optimaux et donc une qualité d'impression perfectible, soit de nombreux réglages sur la machine, et donc un coût d'impression élevé par suite de l'immobilisation du matériel.

La société hollandaise dite "IGT-Reprotest B.V." a proposé récemment un matériel de laboratoire sous la dénomination A.I.C. 2-5 qui permet de déterminer les conditions optimales d'impression d'un papier ou analogue. Pour l'essentiel, ce dispositif comprend :

- un rouleau doseur tramé interchangeable monté fou autour de son axe ;
- un moyen pour déposer sur ce rouleau de l'encre, notamment de l'encre liquide et retirer l'excès d'encre déposé (racle) ;
- un rouleau porte-cliché, moteur, destiné à recevoir un cliché souple sur partie de sa périphérie et à venir au contact tangentiel avec le rouleau doseur pour entraîner celui-ci en rotation ;
- une molette cylindrique, destinée à recevoir un échantillon du papier à tester et à venir au contact tangentiel avec le cliché encré.

Des systèmes de leviers et de contre-poids assurent tant sur le rouleau doseur que sur la molette, des pressions minimales optimales qui sont requises pour la flexographie. Le cliché, généralement en caoutchouc ou en tout autre polymère souple, n'imprime qu'un aplat, ce qui ne donne pas suffisamment de renseignements sur l'aptitude à l'impression en héliogravure du papier testé.

Dans le brevet français de la Demanderesse FRA-2 171 849, on a décrit un dispositif permettant de tester l'aptitude d'un papier à l'impression héliographique,et commercialisé sous la dénomination "HELIOTEST" (marque déposée). Ce dispositif de laboratoire comprend essentiellement deux rouleaux tangents, l'un moteur destiné à recevoir le papier à tester, l'autre monté fou et interchangeable, destiné à recevoir l'encre dont l'excès est enlevé par une racle. Dans ce dispositif, le rouleau interchangeable présente en outre en surface une empreinte constituée par un ensemble de points en creux régulièrement espacés, de profondeur décroissante. Cette disposition donne d'excellents résultats en héliogravure. En revanche, elle ne peut convenir à la flexographie, car, dans ce procédé, l'impression est réalisée par un cliché en relief et non en creux, sur des papiers et cartons ayant des propriétés différentes.

L'invention pallie ces inconvénients. Elle vise un cliché d'essai pour l'impression flexographie qui soit apte à déterminer facilement et rapidement, et de manière significative l'aptitude des papiers ou analogues à ce type d'impression, qui permette également de déterminer les conditions optimales d'impression, notamment pour le choix du type de rouleau doseur à sélectionner.

Ce cliché souple d'impression, pour tester l'aptitude d'un papier,d'un carton ou d'un matériau en feuille ou analogue, à l'impression flexographique, destiné à être monté sur un dispositif d'impression, du type comprenant :

. un rouleau doseur tramé interchangeable,
. un moyen pour déposer de l'encre liquide sur ledit rouleau doseur et retirer l'excès d'encre déposé ;
. un rouleau porte-cliché, moteur, destiné à recevoir ledit cliché souple et à venir en contact tangentiel avec le dit rouleau doseur, et à tourner autour de son axe pour définir un sens de défilement de l'impresion;
. une molette cylindrique, destinée à recevoir un échantillon à tester, et à venir au contact tangentiel avec le cliché encré;

**se caractérise** en ce que le dit cliché comprend au moins un aplat, et au moins une surface tramée dont la linéature varie de façon continue dans le sens de défilement de l'impression.

En d'autres termes,l'invention consiste à utiliser comme cliché d'essai flexographique en cliché souple comprenant une surface tramée entourée par des aplats, dont la linéature varie de façon continue dans le sens de défilement de l'impression, c'est-à-dire dans le sens de rotation du rouleau moteur porte-cliché.

Comme on le sait, une "surface tramée" désigne un quadrillage en réseau très fin, généralement à mailles carrées, composé de points ou de petits carrés fournissant l'image imprimante. De même, la "linéature" d'une trame désigne un nombre de points par unité de longueur contenus dans une ligne.

Avantageusement, en pratique :
- le cliché comprend un cadre périphérique formant aplat et entourant la surface tramée disposée au centre;
- la surface tramée est constituée par une pluralité de points en relief, régulièrement espacées dans le sens de défilement de l'impression et dans le sens orthogonal, et dont le diamètre varie linéairement d'une bord à l'autre de la surface tramée, dans le sens de défilement de l'impression, le rapport entre la surface des points et la surface de la trame étant constant ;
- les points ont le même diamètre sur la même ligne et ce diamètre décroit d'une ligne à l'autre dans le sens de défilement ;
- la surface tramée a une forme rectangulaire allongée bordée de part et d'autre par un aplat ;
- la surface tramée a une linéature qui varie de façon continue dans le sens de défilement entre les valeurs de douze lignes par centimètre jusqu'à cinquante cinq lignes par centimètre, de préférence vingt-sept lignes par centimètre jusqu'à quarante cinq lignes par centimètre ;
- le rapport entre la surface des points et la surface totale tramée est constant et est compris entre dix et soixante dix pourcent (10 et 70 %) et de préférence voisin de quinze à vingt pourcent (15 à 20 %).

La manière dont l'invention peut être réalisée et les avantages qui en découlent ressortiront mieux des exemples de réalisation qui suivent, à l'appui des figures annexées.

La figure 1 est une représentation en vue perspective sommaire d'un appareil de laboratoire commercialisé par IGT Reprotest B.V. sous la dénomination AIC 2-5, muni d'un ensemble d'impression flexographique avec système de dosage de l'encre par molette tramée raclée.

La figure 2 est une vue en coupe sommaire de ce dispositif.

La figure 3 est une représentation du cliché souple caractéristique de l'invention.

La figure 4 est une vue en détail de la linéature caractéristique de ce cliché, alors que la figure 5 montre une variante de cette linéature.

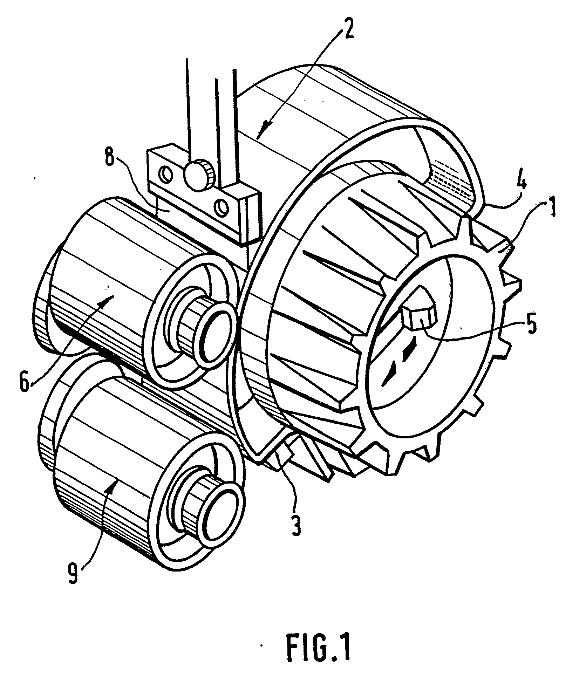

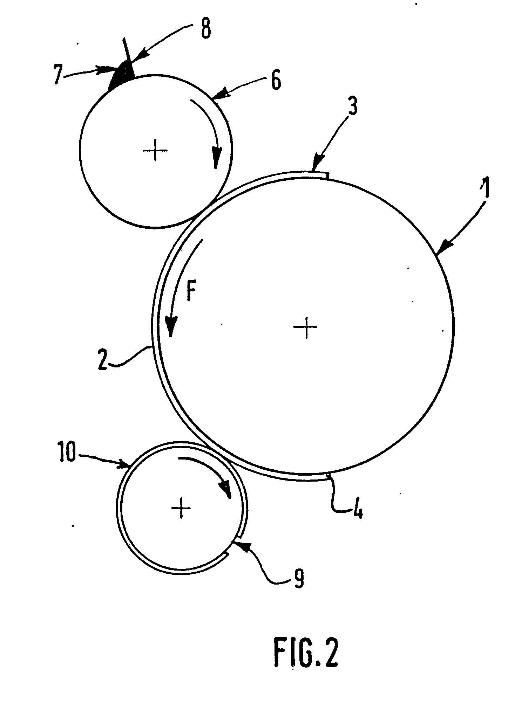

Le dispositif de laboratoire montré aux figures 1 et 2 comprend essentiellement :
. un premier rouleau cylindrique moteur (1) porte-cliché sur lequel est positionné le cliché souple (2) d'impression flexographique entourant sur 180° environ ledit rouleau (1) ; le cliché est maintenu à ses deux extrémités par des mâchoires (3,4) que l'on peut actionner en (5) ; ce rouleau tourne dans le sens indiqué par la flèche F sur la figure 2 (sens de défilement de l'impression) ;
. un second rouleau doseur (6) interchangeable, monté fou sur un axe parallèle à l'axe de rotation du rouleau moteur (1) ; la surface de ce rouleau cylindrique (6) est en métal ou en céramique et est tramée d'une multitude d'alvéoles gravées mais de linéature constante comprise avantageusement entre soixante et trois cents lignes par centimètre, dans lesquelles vient se loger l'encre liquide ; cette gravure est effectuée de manière connue et selon le matériau soit mécaniquement, soit chimiquement, voire même au moyen d'un laser ;
. un moyen pour déposer l'encre liquide (7) sur le rouleau doseur (6) et enlever l'excès d'encre, à savoir une racle (8) en acier, dont le bord utile est disposé parallèlement à la génératrice du rouleau (6) et frotte sur celui-ci ; de la sorte, l'encre (7) pénètre dans les alvéoles gravées dans le rouleau doseur (6), puis est ensuite reportée sur le cliché souple (5) qui, pendant la rotation du rouleau moteur (1) est au contact tangentiel étroit, mais sans pression, avec le rouleau doseur (6) de manière à faire tourner ce dernier ;
. une molette cylindrique (9) destinée à recevoir l'échantillon de papier (ou autre) (10) à tester ; cette molette (9) interchangeable est montée folle autour de son axe parallèle à l'axe de rotation du rouleau (1) et vient au contact étroit tangentiel mais sans pression, avec le cliché encré (2) pendant la rotation du rouleau (1) ; la feuille de papier (10) est fixée sur la molette (9) par tout moyen approprié, tel que machoire, bande adhésive, etc. ; en pratique, il suffit que la surface recto de la feuille (10) à tester affleure la surface du cliché (2) mais sans pression ; dans une forme d'exécution pratique non représentée, on peut, dans la surface de la molette (9), ménager une petite gorge dans laquelle on loge la feuille (10) dont on règle la hauteur par tout moyen connu, tel que notamment des cales, pour obtenir l'affleurement souhaité.

La cliché (2) caractéristique de l'invention peut être réalisé en toute matière appropriée connue pour la fabrication de ces clichés flexographiques, tels que caoutchouc, polymères synthétiques. La gravure de ce cliché s'effectue également par tout moyen connu, tel que photocomposition, moulage, etc. .

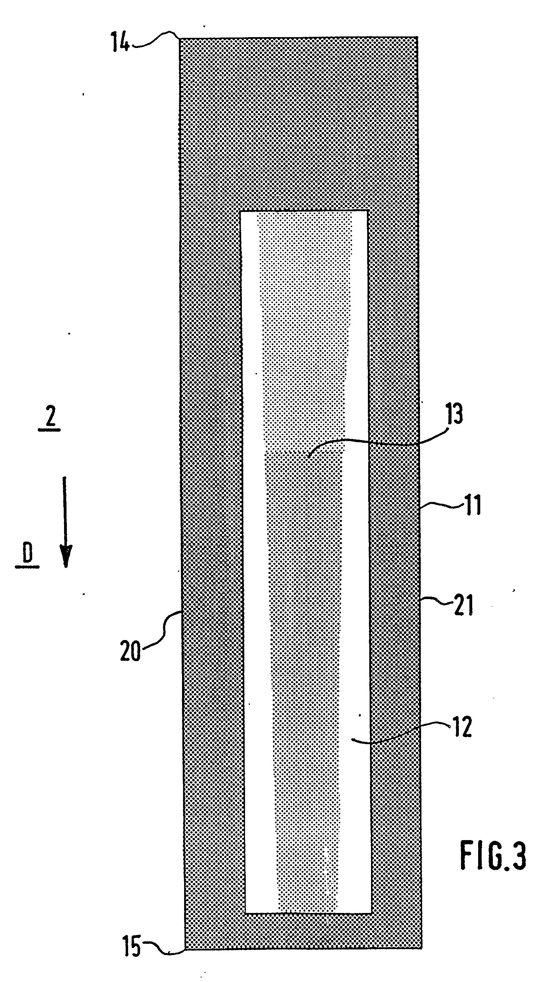

Ce cliché caractéristique (2) montré en détail aux figures 3 à 5 comprend essentiellement un cadre périphérique (11) formant aplat et une portion rectangulaire centrale allongée (12). Cette portion centrale rectangulaire caractéristique (12) présente une trame (13) trapézoïdale dont la linéature varie de façon continue d'un bord (14) à l'autre (15). De préférence, cette linéature décroit dans le sens de la marche, c'est-à-dire dans le sens de défilement de la pression symbolisé par le flèche D.

La trame caractéristique (13) est constituée par une pluralité de points élémentaires (16-19), régulièrement disposés sur des lignes parallèles orthogonales au sens de défilement D. Selon l'invention:
- d'une part, ces points (16-19) ont le même diamètre (ou la même diagonale si le point au lieu d'être circulaire est carré) sur la même ligne, et grossissent d'une ligne à l'autre dans le sens de défilement D ;
- d'autre part, la linéature décroit régulièrement du bord (14) jusqu'au bord (15), avantageusement entre des valeurs comprises entre douze lignes par centimètre en (15,16) jusqu'à cinquante cinq lignes par centimètre (14,17), de préférence entre vingt sept lignes par centimètre jusqu'à quarante cinq lignes par centimètre ;
- enfin, le pourcentage de surface couverte par les points (16-19) est constant quelle que soit la valeur de la linéature, ce rapport étant voisin de quinze pourcent (15 %).

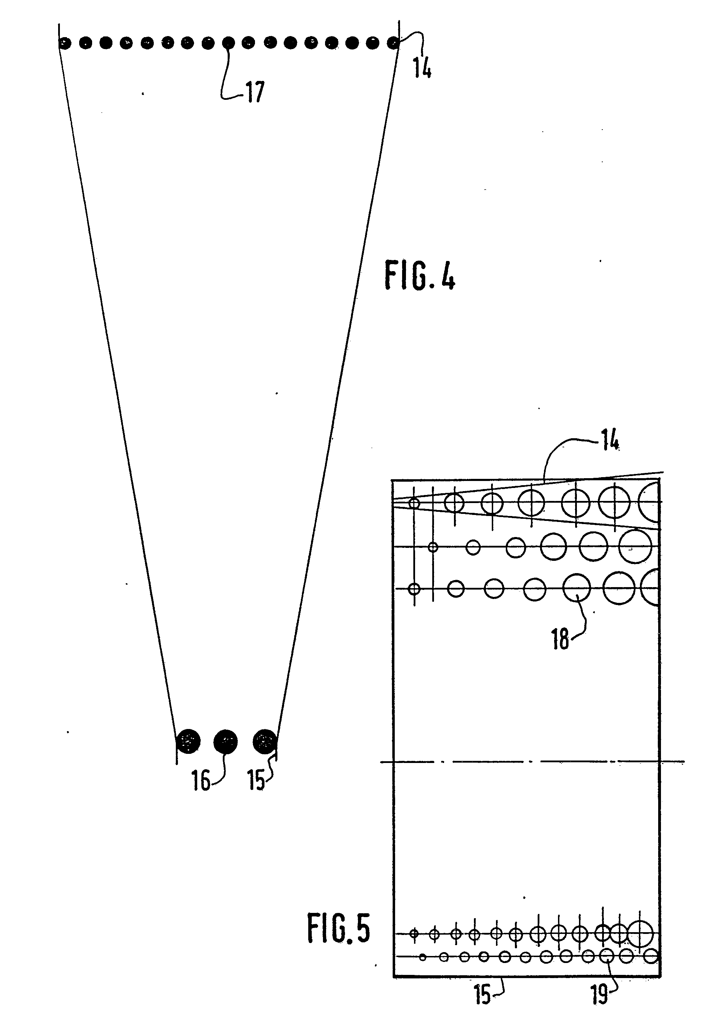

Si dans la forme d'exécution montrée sur les figures 3 et 4, la trame (13) a la forme d'un trapèze inscrit dans un rectangle allongé (12), il va de soi que cette trame peut avoir des formes très variées et, par exemple, occuper toute la surface disponible de ce rectangle (12).

Les aplats latéraux (20,21) disposés de part et d'autre de la trame caractéristique (13) ont la même largeur et servent alors de bandes d'entraînement de la feuille (10) lors de l'impression.

Dans une forme d'exécution pratique, la trame (13) montrée à la figure 3 comprend dans l'ordre :
-une première trame fine, située le plus près possible du bord (14), constituée de soixante points circulaires de 0,1 millimètre de diamètre, régulièrement espacés de 0,13 millimètre sur une largeur de 13,5 millimètres, ce qui donne un linéature de quarante cinq lignes par centimètre et une surface couverte théorique voisine de 14,8 % ;
- à l'autre extrémité, une trame grossière située le plus près possible du bord opposé (15), comprenant vingt points circulaires de 0,166 millimètre de diamètre, régulièrement espacés de 0,115 millimètre sur une largeur de 7,5 millimètres, ce qui donne une linéature de vingt-sept lignes par centimètre et une surface couverte théorique voisine de 14,9 %.

En d'autres termes, quelle que soit la valeur de la linéature, la surface couverte est sensiblement constante et est voisine de quinze pour cent (15 %).

L'utilisation d'outils modernes de tracé graphique permet de produire des variations régulières de la dimension des points, tant en décalant chaque ligne de points, ou chaque ensemble de lignes de points, d'un demi-espace par rapport à la ligne précédente, ou à l'ensemble de lignes précédent.

La bande de papier échantillon (10) à tester, enroulée sur la molette (9), est imprimée par le cliché caractéristique (2). On reproduit donc sur la bande (10) la trame (13) et les aplats (20,21) du cliché (2). Ainsi, pour chaque échantillon (10), on peut apprécier la linéature et le rendu d'aplat qui convient le mieux pour l'impression flexographique de ce papier (10).

La qualité de l'impression de cette bande de papier (10) est un compromis entre la qualité d'impression de l'aplat et celle du tramé. Elle dépend donc essentiellement de la linéature de la trame du rouleau doseur (6). L'évaluation de la qualité d'impression de l'aplat par jugement visuel ou par mesure de réflectance au densitomètre par réflexion su la bande de papier (10), permet donc de sélectionner le rouleau doseur (6) et, partant la linéature convenant le mieux pour effectuer l'encrage du cliché (2). Une fois que l'on a ainsi choisi la linéature du rouleau doseur (6), on vérifie que celle-ci se situe en dehors du risque de moirage. On peut, en évaluant la qualité sur la bande de papier (10) du point de trame de la zone à trame variable, choisir ainsi la linéature de trame convenant le mieux pour imprimer ce papier (10).

Le fait de travailler avec une surface couverte sensiblement constante, par exemple de l'ordre de quinze pour cent (15 %), permet de travailler à densité optique constante et donc de ne pas être influencé par les variations de densité optique, et donc de couleur, pour le choix de la linéature optimale.

Ainsi, le cliché (2) caractéristique de l'invention permet en une seule et même opération de déterminer:
- d'une part, l'aptitude du papier (10) à l'impression flexographique par le choix approprié de la trame ;
- d'ature part, le choix du rouleau doseur de la machine rotative d'impression, afin de ne pas obtenir de moirage et obtenir le meilleur aplat, à savoir par le choix de la profondeur des alvéoles du rouleau doseur pour obtenir la meilleure qualité de points et de linéature.

Dans une autre forme d'exécution montrée à la figure 5, non seulement la linéature de la trame (18,19) décroit de façon continue dans le sens longitudinal (14, 15) et à surface couverte constante, mais également la surface couverte varie de manière progressive dans le sens transversal. De la sorte, la surface couverte dans le sens transversal varie progressivement d'un bord (20) à l'autre (21), par exemple de quinze à soixante dix pourcent (15 à 70 %). Cette réalisation permet de situer avec précision les limites envisageables à la fois en linéature et en surface couverte avec un système donné : papier, encre, rouleau doseur, cliché.

Le cliché selon l'invention permet donc de reproduire sur des échantillons à l'échelle du laboratoire, les conditions industrielles d'impression flexographique. L'imprimeur peut donc ainsi tester différents modes d'impression de papier donné et déterminer les meilleures conditions d'impression par le choix de la trame du rouleau doseur, la linéature de la trame du cliché d'impression et ce, à un coût très réduit par rapport à ce qu'il en coûterait s'il fallait effectuer ces réglages sur la rotative elle-même.

**Revendications**

1/ Cliché souple (2) d'impression, pour tester l'aptitude d'un papier, d'un carton ou d'un matériau analogue en feuille à l'impression flexographique, destiné à être monté sur un dispositif d'impression du type comprenant :
. un rouleau doseur (6) tramé, interchangeable,
. un moyen (8) pour déposer de l'encre liquide (7) sur ledit rouleau (6) et retirer l'excès d'encre (7) déposé ;
. un rouleau porte-cliché (1) moteur, destiné à recevoir ledit cliché (2) et à venir au contact tangentiel avec le dit rouleau doseur (6), et à tourner autour de son axe pour définir un sens de défilement F de l'impresion ;
. une molette cylindrique (9), destinée à recevoir un échantillon (10) à tester, et à venir au contact tangentiel avec le cliché (2) encré ;
**caractérisé** en ce que le dit cliché (2) comprend au moins un aplat (20,21), et au moins une surface tramée (13) dont la linéature varie de façon continue dans le sens de défilement FD de l'impression.

2/ Cliché selon la revendication 1, caractérisé en ce qu'il comporte un cadre périphérique (20,21) formant aplat et entourant la surface tramée (13) disposée au centre dans un rectangle (12) allongé.

3/ Cliché selon l'une des revendications 1 et 2, caractérisé en ce que la surface tramée (13) est constituée par une pluralité de points en relief (16-19), régulièrement espacés dans le sens de défilement FD de l'impression et dans le sens orthogonal à ce sens, et dont le diamètre varie linéairement et progressivement d'un bord (14) à l'autre (15) de la surface tramée (13) dans le sens de défilement de l'impression, le rapport entre la surface des points et la surface de la trame étant constant.

4/ Cliché selon la revendication 3, caractérisé en ce que les points (16-17) ont le même diamètre sur la même ligne et ont un diamètre qui croit d'un ligne à l'autre dans le sens de défilement FD.

5/ Cliché selon la revendication 3, caractérisé en ce que le diamètre des points (18,19) croit d'une ligne à l'autre dans le sens de défilement FD, et également sur la même ligne.

6/ Cliché selon l'une des revendications 1 à 5, caractérisé en ce que la surface tramée a une linéature qui varie de façon continue dans le sens de défilement D entre les valeurs de douze lignes par centimètres (15) jusqu'à cinquante cinq lignes par centimètres (14), de préférence vingt-sept lignes par centimètres (15) jusqu'à quarante cinq lignes par centimètre (14).

7/ Cliché selon la revendication 6, caractérisé en ce que le rapport entre la surface des points (15-17) de la surface tramée (13) et cette surface tramée (13) est constant et est voisin de quinze pour cent (15 %).

FIG.1

FIG.2

FIG.3

FIG.4

17

14

16  15

FIG.5

14

18

15  19

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | FR-A-2 171 849 (CENTRE TECHNIQUE DE L'INDUSTRIE DES PAPIERS, CARTONS ET CELLULOSES) * En entier * | 1,2,6,7 | B 41 F 5/20 G 01 N 33/34 |
| Y | GB-A-1 560 935 (TIMMINS AND SONS) * En entier * | 1,2,6,7 | |
| A | US-A-4 672 893 (MAMMARELLA) * En entier * | 1,4-7 | |

DOMAINES TECHNIQUES
RECHERCHES (Int. Cl.4)

B 41 F
B 41 M
G 01 N

Le présent rapport a été établi pour toutes les revendications

| Lieu de la recherche | Date d'achèvement de la recherche | Examinateur |
|---|---|---|
| LA HAYE | 18-01-1989 | EVANS A.J. |

EPO FORM 1503 03.82 (P0402)